Europäisches Patentamt

European Patent Office

Office européen des brevets

(19)

(11) **EP 1 417 194 B1**

(12) **EUROPÄISCHE PATENTSCHRIFT**

(45) Veröffentlichungstag und Bekanntmachung des Hinweises auf die Patenterteilung:
**16.03.2005 Patentblatt 2005/11**

(21) Anmeldenummer: **02754895.7**

(22) Anmeldetag: **17.07.2002**

(51) Int Cl.$^7$: **C07D 307/60**

(86) Internationale Anmeldenummer:
**PCT/EP2002/007935**

(87) Internationale Veröffentlichungsnummer:
**WO 2003/014100 (20.02.2003 Gazette 2003/08)**

(54) **VERFAHREN ZUR HERSTELLUNG VON MALEINSÄUREANHYDRID**

METHOD FOR PRODUCING MALEIC ANHYDRIDE

PROCEDE DE PREPARATION D'ANHYDRIDE D'ACIDE MALEIQUE

(84) Benannte Vertragsstaaten:
**AT BE BG CH CY CZ DE DK EE ES FI FR GB GR IE IT LI LU MC NL PT SE SK TR**

(30) Priorität: **01.08.2001 DE 10137534**

(43) Veröffentlichungstag der Anmeldung:
**12.05.2004 Patentblatt 2004/20**

(73) Patentinhaber: **BASF AKTIENGESELLSCHAFT 67056 Ludwigshafen (DE)**

(72) Erfinder:
• **HIBST, Hartmut**
  **69198 Schriesheim (DE)**
• **NOE, Ralf**
  **68163 Mannheim (DE)**
• **EXNER, Kai, Michael**
  **69214 Eppelheim (DE)**
• **DUDA, Mark**
  **67071 Ludwigshafen (DE)**

(56) Entgegenhaltungen:
DE-A- 2 241 918          DE-A- 2 539 106
DE-A- 2 603 770          US-A- 4 093 635

# EP 1 417 194 B1

**Beschreibung**

Die vorliegende Erfindung betrifft ein Verfahren zur Herstellung von Maleinsäureanhydrid durch heterogenkatalytische Gasphasenoxidation eines n-Buten enthaltenden Kohlenwasserstoffstroms mit Sauerstoff enthaltenden Gasen in einem Rohrbündelreaktor mit zwei aufeinanderfolgenden Reaktionszonen, wobei die erste, eduktseitige Reaktionszone mindestens einen Katalysator enthält, welcher geeignet ist für die Oxidehydrierung der n-Butene zu 1,3-Butadien und die zweite, produktseitige Reaktionszone mindestens einen Katalysator enthält, welcher geeignet ist für die Oxidation von 1,3-Butadien zu Maleinsäureanhydrid.

Maleinsäureanhydrid ist ein wichtiges Zwischenprodukt bei der Synthese von γ-Butyrolacton, Tetrahydrofuran und 1,4-Butandiol, welche ihrerseits als Lösungsmittel eingesetzt werden oder beispielsweise zu Polymeren, wie Polytetrahydrofuran oder Polyvinylpyrrolidon weiterverarbeitet werden.

Die Herstellung von Maleinsäureanhydrid durch einstufige heterogenkatalytische Gasphasenoxidation von n-Butenen oder n-Butan mit Sauerstoff in Gegenwart eines Vanadium-, Phosphor- und Sauerstoff-enthaltenden Katalysators ist allgemein bekannt und beispielsweise in Ullmann's Encyclopedia of Industrial Chemistry, 6th edition, 1999 Electronic Release, Chapter "MALEIC AND FUMARIC ACID - Maleic Anhydride" beschrieben. Im Falle der Gasphasenoxidation von n-Butan mit Luft wird dabei bei einem n-Butan-Umsatz von 85 % eine Maleinsäureanhydrid-Selektivität von ca. 60 % erreicht (siehe E. Bordes, Catal. Today 16, 1993, Seiten 27 bis 38).

Nachteilig am Einsatz von n-Butan ist dessen oft unzureichende Verfügbarkeit am Standort der Anlage und der dadurch bedingte logistische Aufwand. Deshalb sind als Ausgangsstoff für die wirtschaftliche Herstellung von Maleinsäureanhydrid auch n-Butene, die beispielsweise in reichlichem Maße im $C_4$-Schnitt eines Steamcrackers vorhanden sind, von Interesse. Bei der genannten Gasphasenoxidation von n-Butenen an den genannten Vanadium-, Phosphorund Sauerstoff-enthaltenden Katalysatoren ist die Ausbeute an Maleinsäureanhydrid auf Werte um die 50 Mol-% begrenzt. Von E. Bordes wird bei einem 1-Buten-Umsatz von 95% eine erzielbare Maleinsäureanhydrid-Selektivität von 50 Mol-% angegeben (siehe E. Bordes, Catal. Today 16, 1993, Seiten 27 bis 38). Darüber hinaus werden bei der Selektivoxidation von n-Butenen zu Maleinsäureanhydrid im Vergleich zur n-Butan-Selektivoxidation zusätzlich eine Reihe unerwünschter Nebenprodukte gebildet.

Als weiterer Einsatzstoff für die Herstellung von Maleinsäureanhydrid ist 1,3-Butadien bekannt. So ist in der DE-Offenlegungsschrift 2 241 918 die Gasphasenoxidation von 1,3-Butadien zu Maleinsäureanhydrid in Gegenwart von Molybdän-, Antimon- und Sauerstoff-enthaltenden Katalysatoren beschrieben. Nachteilig am direkten Einsatz von reinem 1,3-Butadien ist dessen unzureichende freie Verfügbarkeit und sein hoher Preis.

Eine kostengünstige Möglichkeit zur Herstellung eines 1,3-Butadien-haltigen Stromes bietet die Oxidehydrierung von n-Butenen oder n-Buten-haltigen Strömen. So ist in A. Yoshioka et al., Hydrocarbon Processing, 63, November 1984, Seite 97 bis 100 die Oxidehydrierung eines Raffinat-II-Stromes mit einer 1,3-Butadien-Ausbeute von 78 % beschrieben.

Die DE-Offenlegungsschrift 26 03 770 beschreibt die Herstellung von Maleinsäureanhydrid durch Umsetzung von n-Butenen mit Luft in einem Wirbelschichtreaktor in Gegenwart eines Oxidationskatalysators, welcher in der Oxidehydrierung der n-Butene zu 1,3-Butadien wirksam ist, und eines Oxidationskatalysators, welcher in der Oxidation von 1,3-Butadien zu Maleinsäureanhydrid wirksam ist. Nachteilig an diesem Verfahren ist eine geringe Maleinsäureanhydrid-Ausbeute von 30 %.

Die DE-Offenlegungsschrift 25 39 106 lehrt die Herstellung von Maleinsäureanhydrid durch Umsetzung von n-Butan und n-Buten-haltigen Strömen mit Luft in einem Rohrbündelreaktor mit einer ungeteilten, isotherm temperierten Reaktionszone, welche zwei unterschiedliche Katalysatoren enthält. Der auf der Eduktseite vorliegende Katalysator oxidehydriert n-Butan und n-Buten zu 1,3-Butadien. Der auf der Produktseite vorliegende Katalysator oxidiert das gebildete 1,3-Butadien zu Maleinsäureanhydrid. Unter Einsatz eines Oxidehydrierkatalysators auf Basis von Bismutmolybdat und eines Oxidationskatalysators auf Basis von Antimonmolybdat wurde durch Einsatz eines cis- und trans-2-Buten-Gemischs eine Maleinsäureanhydrid-Ausbeute von 60 % erreicht.

Erfindungsgemäß wurde erkannt, dass die beschriebene direkte Hintereinanderschaltung von Oxidehydrierung und Oxidation und insbesondere der isotherme Betrieb beider Prozesse viele Nachteile nach sich zieht. Es wurde des weiteren erfindungsgemäß erkannt, dass für den optimalen Betrieb der Oxidehydrierung von n-Butenen zu 1,3-Butadien sowie der Oxidation von 1,3-Butadien zu Maleinsäureanhydrid sehr unterschiedliche Reaktionstemperaturen erforderlich sind. Darüber hinaus unterscheiden sich die Wärmetönungen beider Reaktionsschritte sehr deutlich. Während bei der Oxidehydrierung der n-Butene zu 1,3-Butadien nur etwa 130 kJ/Mol freigesetzt werden, liefert die Oxidation von 1,3-Butadien zu Maleinsäureanhydrid etwa 990 kJ/Mol. Eine an die Exothermie angepasste Wärmeabfuhr ist somit faktisch nicht möglich. Des weiteren ist eine Anpassung an die optimalen Betriebsbedingungen der beiden Katalysatoren ebenfalls nicht oder nur äußerst bedingt möglich. So können Effekte durch unterschiedliche Katalysatoraktivitäten, durch unterschiedlich stark fortschreitende Desaktivierungsprozesse, durch Schwankungen in der Reinheit und Qualität des Eduktstroms oder auch durch sogenannte Last-Änderungen (Änderungen der Strömungsgeschwindigkeit und/oder der Eduktzufuhr) nicht oder nur stark eingeschränkt korrigiert werden.

Es bestand daher die Aufgabe, ein Verfahren zur Herstellung von Maleinsäureanhydrid durch heterogenkatalytische Gasphasenoxidation eines preiswerten und gut verfügbaren Kohlenwasserstoffstroms mit Sauerstoff zu entwickeln, welches die oben genannten Nachteile nicht besitzt und bei hoher Kohlenwasserstoff-Belastung des Katalysators einen hohen Umsatz, eine hohe Selektivität sowie eine hohe Ausbeute an Wertprodukt und somit eine hohe Raum-Zeit-Ausbeute ermöglicht. Eine weitere Aufgabe der vorliegenden Erfindung war es, eine flexible Reaktionsführung zu ermöglichen, welche auch bei Schwankungen in der Menge, Qualität oder Reinheit der Ausgangsstoffe oder bei einer fortschreitenden Katalysatordesaktivierung eine hohe Raum-Zeit-Ausbeute über einen langen Zeitraum hinweg ermöglicht.

Demgemäß wurde ein Verfahren zur Herstellung von Maleinsäureanhydrid durch heterogenkatalytische Gasphasenoxidation eines n-Buten enthaltenden Kohlenwasserstoffstroms mit Sauerstoff enthaltenden Gasen in einem Rohrbündelreaktor mit zwei aufeinanderfolgenden Reaktionszonen, wobei die erste, eduktseitige Reaktionszone mindestens einen Katalysator enthält, welcher geeignet ist für die Oxidehydrierung der n-Butene zu 1,3-Butadien und die zweite, produktseitige Reaktionszone mindestens einen Katalysator enthält, welcher geeignet ist für die Oxidation von 1,3-Butadien zu Maleinsäureanhydrid, das dadurch gekennzeichnet ist, dass man einen Rohrbündelreaktor einsetzt, welcher mindestens einen Wärmeträgerkreislauf im Bereich der ersten, eduktseitigen Reaktionszone und mindestens einen weiteren Wärmeträgerkreislauf im Bereich der zweiten, produktseitigen Reaktionszone aufweist.

Unter dem Begriff Rohrbündelreaktor ist ein Reaktor zu verstehen, welcher mindestens ein Reaktorrohr, welches zur Beheizung und/oder Kühlung von einem Wärmeträgermedium umgeben ist, enthält. Im Allgemeinen enthalten die technisch eingesetzten Rohrbündelreaktoren wenige hundert bis mehrere zehntausend parallel-geschaltete Reaktorrohre. Sind mehrerer einzelne Rohrbündelreaktoren (im Sinne von Rohrbündelreaktor-Apparaten) parallel-geschaltet, so sind diese als Äquivalent eines Rohrbündelreaktors zu verstehen und im Folgenden durch den Begriff Rohrbündelreaktor mit umfasst.

Unter dem Begriff erste, eduktseitige Reaktionszone ist der Bereich innerhalb des Rohrbündelreaktors zu verstehen, welcher mindestens einen Katalysator enthält, welcher geeignet ist für die Oxidehydrierung der n-Butene zu 1,3-Butadien. Unter dem Begriff zweite, produktseitige Reaktionszone ist der Bereich innerhalb des Rohrbündelreaktors zu verstehen, welcher mindestens einen Katalysator enthält, welcher geeignet ist für die Oxidation von 1,3-Butadien zu Maleinsäureanhydrid.

Wesentlich beim erfindungsgemäßen Verfahren ist, dass man einen Rohrbündelreaktor einsetzt, welcher einen Wärmeträgerkreislauf im Bereich der ersten, eduktseitigen Reaktionszone und einen weiteren Wärmeträgerkreislauf im Bereich der zweiten, produktseitigen Reaktionszone aufweist. So kann beispielsweise jede der beiden Reaktionszonen durch einen, zwei, drei oder mehrere Wärmeträgerkreisläufe temperiert sein. Bevorzugt ist der Einsatz eines Rohrbündelreaktors, welcher im Bereich der ersten und im Bereich der zweiten Reaktionszone jeweils genau einen Wärmeträgerkreislauf aufweist.

Als Rohrbündelreaktoren können beim erfindungsgemäßen Verfahren im Prinzip alle bekannten Rohrbündelreaktoren mit zwei oder mehreren Wärmeträgerkreisläufen eingesetzt werden. Bevorzugt ist der Einsatz von Rohrbündelreaktoren mit zwei Wärmeträgerkreisläufen. Geeignete Rohrbündelreaktoren sind beispielsweise in US 3,147,084, DE-C 28 30 765, EP-A 0 911 313 und EP-A 1 097 745 beschrieben.

Im Allgemeinen weisen die beim erfindungsgemäßen Verfahren einsetzbaren Rohrbündelreaktoren einen äußeren Reaktorkorpus mit einem oberen und unteren Anschluss für die Zu- und Abfuhr der Reaktionsgase auf. Die Reaktorrohre sind am oberen und unteren Ende mit einer oberen und unteren Röhrenplatten verschweißt. Der die Reaktorrohre umgebende Raum zwischen der oberen und unteren Röhrenplatte ist in der Regel durch eine Trennplatte in zwei Zonen unterteilt. Jede der zwei Zonen weist am Reaktorkorpus üblicherweise zwei Anschlüsse für die Zu- und Abfuhr des Wärmeträgermediums auf. Im Allgemeinen befindet sich die Trennplatte auf der Höhe des Übergangsbereichs zwischen den beiden aufeinanderfolgenden Reaktionszonen.

In einer Ausgestaltungsform kann die Trennplatte, wie in US 3,147,084 beschrieben, mit den Reaktorrohren fest verbunden sein und die beiden Wärmeträgerzonen gegeneinander abdichten.

In einer anderen Ausgestaltungsform kann die Trennplatte zu den einzelnen Reaktorrohren jeweils einen möglichst geringen Spalt aufweisen, welcher eine ungehinderte Wärmeausdehnung der Reaktorrohre ermöglicht und somit mechanische Spannungen und eine dadurch induzierbare Korrosion und mechanische Schädigungen verhindert. Eine derartige Konstruktion ist beispielsweise in DE-C 28 30 765 beschrieben. In einer anderen Ausgestaltungsform sind die Reaktorrohre im Bereich der Trennplatte in Richtung der Trennplatte aufgedehnt um den Spalt zur Trennplatte weiter zu verkleinern. Die Durchbohrung der Trennplatte kann dabei auf ihrer Innenseite zur Verbesserung der Abdichtung der beider Wärmeträgermedien mit zusätzlichen Rillen versehen sein, wie dies in der EP-A 1 097 745 beschrieben ist. Es ist auch möglich, dass die Trennplatte zwar fest mit den Reaktorrohren verbunden ist, jedoch einen Ringspalt zum Reaktorkorpus hin aufweist. Um mechanische Spannungen durch ungleichmäßige Wärmeausdehnung zu vermeiden, kann der Reaktorkorpus auch mit sogenannten Expansionszonen, beispielsweise in Form halbrunder Ausbuchtungen, ausgerüstet sein. Ein derartig konstruierter Reaktor ist beispielsweise in EP-A 1 097 745 beschrieben.

Üblicherweise sind in den vorgenannten Rohrbündelreaktoren die Reaktorrohre aus ferritischem Stahl gefertigt und weisen in typischer Weise eine Wanddicke von 1 bis 3 mm auf. Ihr Innendurchmesser beträgt in der Regel 15 bis 30 mm. Die Anzahl der Reaktorrohre je Rohrbündelreaktor liegt üblicherweise im Bereich zwischen 5 000 und 35 000, obgleich auch eine Anzahl über 35 000 bei besonders großen Anlagen realisiert werden kann. Innerhalb des Reaktorkorpus sind die Reaktorrohre im Normalfall homogen verteilt angeordnet.

Die Wärmeträgerzonen können des weiteren zur gezielten Lenkung des Wärmeträgermediums diverse Einbauten enthalten. Bevorzugt sind Einbauten, welche eine Umlenkung zumindest eines Teils des zirkulierenden Wärmeträgermediums in radialer Richtung, d. h. innerhalb der Querschnittsfläche, fördern und somit weitgehend eine möglichst senkrechte, d. h. radiale Anströmung der in axialer Richtung verlaufenden Reaktorrohre ermöglichen. Bevorzugt sind Einbauten, welche einen im Wesentlichen mäanderförmigen Strömungsverlauf zumindest eines Teils des Wärmeträgermediums fördern. Explizit genannt sei dabei eine alternierende Anordnung von "donut-förmigen" Ablenkplatten mit einem mittig angeordneten Strömungsloch und kreisrunden Ablenkplatten mit einem Strömungsspalt zwischen Reaktorkorpus und Ablenkplatte, wie beispielsweise in EP-A 1 097 745 beschrieben.

Als Wärmeträgermedien eignen sich insbesondere fluide Temperiermedien. Besonders günstig ist die Verwendung von Salzschmelzen, wie Kaliumnitrat, Kaliumnitrit, Natriumnitrat und/oder Natriumnitrit oder von niedrig schmelzenden Metallen wie Natrium sowie Legierungen verschiedener Metalle. Bevorzugt ist die Verwendung von Salzschmelzen.

Die Strömungsrichtung des Wärmeträgermediums im Reaktor kann prinzipiell von oben nach unten oder von unten nach oben erfolgen, wobei diese für die beiden Reaktionszonen unabhängig voneinander gewählt werden kann. Aus strömungstechnischen Gründen, vor allem wegen der induzierten Konvektion, ist beim erfindungsgemäßen Verfahren eine Durchströmung des Wärmeträgermediums im Reaktor von unten nach oben bevorzugt.

Die beim erfindungsgemäßen Verfahren einsetzbaren Rohrbündelreaktoren können mit dem Reaktionsgas von oben nach unten als auch von unten nach oben durchströmt werden. Im erstgenannten Fall befindet sich der Katalysator, welcher für die Oxidehydrierung der n-Butene zu 1,3-Butadien geeignet ist in der oberen Reaktionszone und der Katalysator, welcher für die Oxidation von 1,3-Butadien zu Maleinsäureanhydrid geeignet ist in der unteren Reaktionszone. Im zweitgenannten Fall ist die Anordnung des Katalysators umgekehrt.

Fließt das Wärmeträgermedium in dieselbe Richtung wie das Reaktionsgas, so spricht man von einer Gleichstromfahrweise; fließt das Wärmeträgermedium in die entgegengesetzte Richtung, so spricht man von einer Gegenstromfahrweise. Bevorzugt beim erfindungsgemäßen Verfahren ist die Gleichstromfahrweise, da diese im Allgemeinen eine bessere Verteilung des Heißpunktes (Hotspots) ermöglicht.

Aufgrund der bevorzugten Führung des Wärmeträgermediums von unten nach oben sowie einer Gleichstromfahrweise ist beim erfindungsgemäßen Verfahren eine aufsteigende Durchströmung mit den Reaktionsgasen und den beiden Wärmeträgermedien besonders bevorzugt.

Der einzusetzende Rohrbündelreaktor kann des weiteren auch eine oder mehrere integrierte oder vorgeschaltete Vorheizzonen enthalten, welche das eintretende Gasgemisch aufheizen. Eine in einem Rohrbündelreaktor integrierte Vorheizzone kann beispielsweise durch mit Inertmaterial gefüllte Reaktorrohre, welche ebenfalls von einem Wärmeträgermedium umgeben sind, realisiert werden. Als Inertmaterial sind prinzipiell alle Materialien geeignet, welche nicht zur chemischen Umsetzung des durchströmenden Reaktionsgases beitragen, d. h. keine heterogenkatalytische Reaktion induzieren oder katalysieren, und welche einen maximalen Druckverlust unterhalb des jeweiligen, maximal tolerierbaren, anlagenspezifischen Wertes aufweisen. Geeignet sind beispielsweise oxidische Materialien, wie etwa $Al_2O_3$, SiC oder metallische Materialien, wie etwa Edelstahl. Das Inertmaterial kann beispielsweise in Form von Formkörpern, Netzen, offenen Schwämmen und Gewirken oder Einbauten, wie sie üblicherweise auch bei statischen Mischern eingesetzt werden, vorliegen. Bevorzugt sind Formkörper wie beispielsweise Kugeln, Tabletten, Hohlzylinder, Ringe, Trilobes, Tristars, Wagenräder, Extrudate oder regellos gebrochene Formkörper.

Beim erfindungsgemäßen Verfahren können die Katalysatoren der zwei aufeinanderfolgenden Reaktionszonen beispielsweise direkt aufeinanderfolgen oder durch einen Leerraum oder eine Zwischenschicht voneinander getrennt sein. Der Übergang der beiden Katalysatoren, der Leerraum sowie die Zwischenschicht befinden sich jeweils bevorzugt etwa auf der Höhe oder in der Nähe der Trennplatte.

Bevorzugt leitet man beim erfindungsgemäßen Verfahren das Reaktionsgas aus der ersten, eduktseitigen Reaktionszone vor Eintritt in die zweite, produktseitige Reaktionszone durch eine inerte Zwischenschicht. Als inerte Materialien für die genannte Zwischenschicht sind im Allgemeinen Materialien geeignet, welche unter den vorliegenden Reaktionsbedingungen im Wesentlichen nicht zur chemischen Umsetzung des durchströmenden Reaktionsgases beitragen, d. h. keine heterogenkatalytische Reaktion induzieren oder katalysieren, und welche einen maximalen Druckverlust unterhalb des jeweiligen, maximal tolerierbaren, anlagenspezifischen Wert aufweisen. Geeignet sind beispielsweise oxidische Materialien, wie etwa $Al_2O_3$, SiC oder metallische Materialien, wie etwa Edelstahl. Das Inertmaterial kann beispielsweise in Form von Formkörpern, Netzen, offenen Schwämmen und Gewirken oder Einbauten, wie sie üblicherweise auch bei statischen Mischern eingesetzt werden, vorliegen. Bevorzugt sind Formkörper wie beispielsweise Kugeln, Tabletten, Hohlzylinder, Ringe, Trilobes, Tristars, Wagenräder, Extrudate oder regellos gebrochene Formkörper. Die maximalen Durchmesser der bevorzugten Formkörper liegen bevorzugt zwischen 1/10 und maximal 1/1 des Reaktorrohrinnendurchmessers.

Je nachdem, ob die zweite Reaktionszone bei höherer oder niedrigerer Temperatur betrieben wird, ermöglicht die inerte Zwischenschicht eine Aufheizung oder Abkühlung des Reaktionsgases. Um diesen Zweck zu erfüllen, befindet sich der überwiegende Teil der inerten Zwischenschicht in der Regel im Bereich der zweiten, heißeren bzw. kälteren Zone. Des weiteren ermöglicht die inerte Zwischenschicht eine Abscheidung höhermolekularer Nebenprodukte aus der ersten Reaktionszone und wirkt einer Ablagerung am Katalysator der zweiten Reaktionszone und somit einem allmählichen Anstieg des Druckverlustes durch derartige Ablagerungen am Katalysator entgegen.

Die Länge der Zwischenschicht ist bevorzugt so zu bemessen, dass der wesentliche Teil der Temperaturdifferenz zwischen beiden Reaktionszonen überwunden wird. Die Länge der Zwischenschicht ist gegebenenfalls auch nach der Menge der gebildeten höhermolekularer Nebenprodukte der ersten Reaktionszone und dem gewünschten Grad der Entfernung dieser Nebenprodukte zu wählen. Diese kann beispielsweise durch einfache Labor- oder Technikumsversuche ermittelt werden.

Das inerte Material der inerten Zwischenschicht weist bevorzugt ein Leerraumvolumen von 40 bis 99,5% auf, wobei das Leerraumvolumen definiert ist als: Leerraumvolumen = [(Gesamtvolumen) - (geometrisches Volumen)] / (Gesamtvolumen). Unter dem "Gesamtvolumen" ist das gesamte Volumen der inerten Zwischenschicht im Reaktionsrohr zu verstehen. Das "geometrische Volumen" gibt das makroskopische Festkörpervolumen der inerten Materialien wieder, wobei eventuell vorhandene Poren innerhalb des inerten Materials zum geometrischen Volumen hinzugerechnet werden. Bei einem Leerraumvolumen kleiner 40 % nimmt der Druckverlust in der Regel deutlich zu. Bei einem Leerraumvolumen größer 99,5% ist im Allgemeinen der gewünschte Effekt der Aufheizung oder Abkühlung sowie der Abscheidung höhermolekularer Nebenprodukte unbefriedigend. Bevorzugt beträgt der Leerraumanteil des inerten Materials 45 bis 99 %.

Als Katalysatoren können beim erfindungsgemäßen Verfahren im Prinzip alle für die Oxidehydrierung der n-Butene zu 1,3-Butadien sowie alle für die Oxidation von 1,3-Butadien zu Maleinsäureanhydrid geeigneten Katalysatoren eingesetzt werden.

Die für die Oxidehydrierung der n-Butene zu 1,3-Butadien besonders geeigneten Katalysatoren basieren im Allgemeinen auf einem Mo-Bi-O-haltigem Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Magnesium, Zirkon, Chrom, Nickel, Cobalt, Cadmium, Zinn, Blei, Germanium, Lanthan, Mangan, Wolfram, Phosphor, Aluminium oder Silizium.

Geeignete Katalysatoren und deren Herstellung sind beispielsweise beschrieben in US 4,423,281 ($Mo_{12}BiNi_8Pb_{0,5}Cr_3K_{0,2}O_x$ und $Mo_{12}Bi_bNi_7Al_3Cr_{0,5}K_{0,5}O_x$), US 4,336,409 ($Mo_{12}BiNi_6Cd_2Cr_3P_{0,5}O_x$), DE-A 26 00 128 ($Mo_{12}BiNi_{0,5}SCr_3P_{0,5}Mg_{7,5}K_{0,1}O_x + SiO_2$) und DE-A 24 40 329 ($Mo_{12}BiCo_{4,5}Ni_{2,5}Cr_3P_{0,5}K_{0,1}O_x$), worauf explizit Bezug genommen wird.

Die Stöchiometrie der Aktivmasse einer Vielzahl der für die Oxidehydrierung der n-Butene zu 1,3-Butadien geeigneten Multimetalloxidkatalysatoren läßt sich unter der allgemeinen Formel (I)

$$Mo_{12}Bi_aFe_bCo_cNi_dCr_eX^1{}_fk_gO_x \qquad (I),$$

in der die Variablen nachfolgende Bedeutung aufweisen:

$X^1$ = W, Sn, Mn, La, Ce, Ge, Ti, Zr, Hf, Nb, P, Si, Sb, Al, Cd und/oder Mg;
a = 0,5 bis 5, vorzugsweise 0,5 bis 2;
b = 0 bis 5, vorzugsweise 2 bis 4;
c = 0 bis 10, vorzugsweise 3 bis 10;
d = 0 bis 10;
e = 0 bis 10, vorzugsweise 0,1 bis 4;
f = 0 bis 5, vorzugsweise 0,1 bis 2;
g = 0 bis 2, vorzugsweise 0,01 bis 1; und
x = eine Zahl, die durch die Wertigkeit und Häufigkeit der von Sauerstoff verschiedenen Elemente in (I) bestimmt wird;

subsummieren.

Bevorzugt setzt man beim erfindungsgemäßen Verfahren für die Oxidehydrierung ein Mo-Bi -Fe-O-haltiges Multimetalloxidsystem ein, wobei ein Mo-Bi-Fe-Cr-O- oder Mo-Bi-Fe-Zr-O-haltiges Multimetalloxidsystem besonders bevorzugt ist. Bevorzugt Systeme sind beispielsweise beschrieben in US 4,547,615 ($Mo_{12}BiFe_{0,1}Ni_8ZrCr_3K_{0,2}O_x$ und $Mo_{12}BiFe_{0,1}Ni_8AlCr_3K_{0,2}O_x$), US 4,424,141 ($Mo_{12}BiFe_3Co_{4,5}Ni_{2,5}P_{0,5}K_{0,1}O_x + SiO_2$), DE-A 25 30 959

($Mo_{12}BiFe_3CO_{4,5}Ni_{2,5}Cr_{0,5}K_{0,1}O_x$, $Mo_{13,75}BiFe_3Co_{4,5}Ni_{2,5}Ge_{0,5}K_{0,8}O_x$, $Mo_{12}BiFe_3Co_{4,5}Ni_{2,5}Mn_{0,5}K_{0,1}O_x$ und $Mo_{12}BiFe_3Co_{4,5}Ni_{2,5}La_{0,5}K_{0,1}O_x$). US 3,911,039 ($Mo_{12}BiFe_3Co_{4,5}Ni_{2,5}Sn_{0,5}K_{0,1}O_x$), DE-A 25 30 959 und DE-A 24 47 825 ($Mo_{12}BiFe_3Co_{4,5}Ni_{2,5}W_{0,5}K_{0,1}O_x$). Herstellung und Charakterisierung der genannten Katalysatoren sind ausführlich in den zitierten Schriften beschrieben, auf die hiermit explizit verwiesen wird.

Die für die Oxidation von 1,3-Butadien zu Maleinsäureanhydrid geeigneten Katalysatoren basieren im Allgemeinen auf einem Molybdänoxid-haltigem und bevorzugt auf einem im Wesentlichen Mo-Sb-O-haltigem oder Mo-V-O-haltigem Multimetalloxidsystem.

Im Falle des Mo-Sb-0-haltigen Multimetalloxidsystems enthält das Katalysatorsystem im Allgemeinen noch weitere Komponenten aus der 1. bis 8. und 14. Gruppe des Periodensystems sowie aus der Reihe der Lanthaniden, wie beispielsweise Lithium, Titan, Vanadium, Niob, Wolfram, Eisen, Zinn und Cer.

Im Falle des Mo-Sb-0-haltigen Multimetalloxidsystems läßt sich die Zusammensetzung der Aktivmasse einer Vielzahl der für die Oxidation von 1,3-Butadien zu Maleinsäureanhydrid geeigneten Multimetalloxidkatalysatoren unter der allgemeinen Formel (II)

$$SbMo_aSn_bX^2{}_cO_x \hspace{3cm} (II),$$

in der die Variablen nachfolgende Bedeutung aufweisen:

$X^2 =$      V, Fe, Ni, Li, Ce, Nb, Ta, W, Ti, Zr, B, P, Al und/oder Mg;

$a =$      1 bis 10, vorzugsweise 2 bis 4;

$b =$      0 bis 5, vorzugsweise 0,01 bis 2;

$c =$      0 bis 3, vorzugsweise 0,01 bis 1,5; und

$x =$      eine Zahl, die durch die Wertigkeit und Häufigkeit der von Sauerstoff verschiedenen Elemente in (II) bestimmt wird;

subsummieren.

Geeignete Katalysatoren und deren Herstellung sind beispielsweise beschrieben in JP-A 05 057 188 ($Sb_2Mo_{10}O_x$ entspricht $SbMo_5O_x$), DE-A 22 41 918 ($Sb_4Mo_{6,12}O_x$ entspricht $SbMo_{1,53}O_x$), DE-A 23 22 186 ($SbMo_3V_{0,1}Fe_{0,2}W_{0,06}O_x$), DE 27 02 606 ($SbMo_3V_{0,1}Li_{0,1}W_{0,06}O_x$ und ($SbMo_3V_{0,1}Ce_{0,1}W_{0,06}O_x$) und DE-A 28 13 424 ($SbMo_{3,06}T_{0,6}N_{0,1}O_x$). Herstellung und Charakterisierung der genannten Katalysatoren sind ausführlich in den zitierten Schriften, auf die hiermit explizit verwiesen wird, beschrieben.

Im Falle des Mo-V-O-haltigen Multimetalloxids läßt sich die Zusammensetzung der Aktivmasse einer Vielzahl der für für die Oxidation von 1,3-Butadien zu Maleinsäureanhydrid geeigneten Multimetalloxidkatalysatoren unter der allgemeinen Formel (III)

$$Mo_{12}V_aW_bX^3{}_cO_x \hspace{3cm} (III),$$

in der die Variablen nachfolgende Bedeutung aufweisen:

$X^3 =$      La, Mn, Fe, Cu, Al, Co, Ni, Bi, Ag, P, Zn, Cd, As, Cr, Sn, U, Ti, Nb, Ge, Alkali und/oder Erdalkali;

$a =$      0,1 bis 12, vorzugsweise 1,5 bis 10;

$b =$      0 bis 5, vorzugsweise 0,1 bis 4;

$c =$      0 bis 12, vorzugsweise 0,1 bis 10; und

$x =$      eine Zahl, die durch die Wertigkeit und Häufigkeit der von Sauerstoff verschiedenen Elemente in (III) bestimmt wird;

subsummieren.

Geeignete Katalysatoren und deren Herstellung sind beispielsweise beschrieben in US 3,893,951 ($Mo_{12}V_3W_{1,2}O_x$, $Mo_{12}V_3W_{1,2}Sn_6O_x$), US 4,157,987 ($Mo_{12}V_3W_{1,2}Ce_3O_x$, $Mo_{12}V_3W_{1,2}Ce_2CoO_x$, $Mo_{12}V_3W_{1,2}Ce_2Cu_2O_x$) DE-A 24 59 092 ($Mo_{12}V_3W_{1,2}U_2O_x$), US 4,170,570 ($Mo_{12}V_3W_{1,2}Cu_2Sn_{0,5}O_x$), US 4,378,309 ($Mo_{12}V_3Cu_{0,5}GeO_x$), US 4,042,533 ($Mo_{12}V_3W_{1,2}Ti_{0,5}O_x$), US 4,115,441 ($Mo_{12}V_3GeFe_{0,1}O_x$), US 4,138,366 ($Mo_{12}V_3SbCd_{0,2}P_{0,1}O_x$) und US 4,250,054 ($Mo_{12}V_3W_{1,2}La_{0,5}Co_{0,1}O_x$). Herstellung und Charakterisierung der genannten Katalysatoren sind ausführlich in den zitierten Schriften beschrieben, auf die hiermit explizit verwiesen wird.

Die beiden Katalysatoren zur Oxidehydrierung und zur Oxidation werden im Allgemeinen als Formkörper mit einer mittleren Größe von über 2 mm eingesetzt. Aufgrund des zu beachtenden Druckverlustes während der Ausübung des Verfahrens sind kleinere Formkörper in der Regel ungeeignet. Als geeignete Formkörper seien beispielsweise genannt Tabletten, Zylinder, Hohlzylinder, Ringe, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Im Allgemeinen können die zu verwendenden Katalysatoren als sogenannte Vollkatalysatoren eingesetzt werden. In diesem Fall besteht der gesamte Katalysatorformkörper aus der Aktivmasse, inklusive eventueller Hilfsmittel, wie etwa Graphit oder Porenbildner, sowie weiterer Komponenten. Insbesondere hat es sich als günstig erwiesen, den für die Oxidehydrierung der n-Butene zu 1,3-Butadien bevorzugt zu verwendenden Mo-Bi-Fe-O-haltigen Katalysator als Vollkatalysator einzusetzen. Des weiteren ist es möglich, die Aktivmassen der Katalysatoren auf einen Träger, beispielsweise einen anorganischen, oxidischen Formkörper, aufzubringen. Derartige Katalysatoren werden in der Regel als Schalenkatalysatoren bezeichnet. Insbesondere hat es sich als günstig erwiesen, den für die Oxidation des im ersten Schritt erzeugten 1,3-Butadiens zu Maleinsäureanhydrid bevorzugt zu verwendenden Mo-Sb-O- bzw. Mo-V-O-haltigen Katalysator als Schalenkatalysator einzusetzen.

Beim erfindungsgemäßen Verfahren werden in den beiden Reaktionszonen unterschiedliche Katalysatoren eingesetzt, welche für die Oxidehydrierung der n-Butene zu 1,3-Butadien beziehungsweise für die Oxidation von 1,3-Butadien zu Maleinsäureanhydrid optimiert sind. Ebenso werden bevorzugt für beide Reaktionszonen die jeweils optimierte Reaktionstemperatur eingestellt.

Bezüglich des Einsatzes der Katalystoren der ersten, eduktseitigen Reaktionszone beziehungsweise der zweiten, produktseitigen Reaktionszone sind beim erfindungsgemäßen Verfahren verschiedene Varianten möglich. In einer Variante enthält jede Reaktionszone eine homogene Katalysatorschüttung, d. h. eine Katalysatorschüttung, welche überall innerhalb der jeweiligen Reaktionszone pro Volumeneinheit im Mittel die gleiche Zusammensetzung und die gleiche Aktivität besitzt. Eine Katalysatorschüttung kann sich zusammensetzen aus Formkörpern des gleichen Katalysators, aus Formkörpern einer Mischung verschiedener Katalysatoren oder auch aus Formkörpern (gleicher Katalysator oder Mischung verschiedener Katalysatoren), welche mit einem Inertmaterial vermischt, d. h. "verdünnt" sind. Als Inertmaterialien kommen prinzipiell alle Formkörper in Betracht, welche auch für den Einsatz in den Vorheizzonen sowie als inerte Zwischenschicht geeignet sind. Auf die obigen Ausführungen wird verwiesen.

In einer anderen Variante enthält die erste, eduktseitige und/oder die zweite, produktseitige Reaktionszone eine heterogene Katalysatorschüttung, d. h. eine Katalysatorschüttung, welche innerhalb der jeweiligen Reaktionszone, abhängig von der Lage innerhalb dieser Reaktionszone, pro Volumeneinheit im Mittel eine unterschiedliche Zusammensetzung und eine unterschiedliche Aktivität besitzt. Die Bereitstellung einer heterogenen Katalysatorschüttung wird im Allgemeinen erreicht durch eine heterogene Mischung verschiedener Katalysatoren oder auch aus Formkörpern (gleicher Katalysator oder Mischung verschiedener Katalysatoren), welche mit einem Inertmaterial heterogen durchmischt, d. h. "verdünnt" sind. Die lokale Aktivität innerhalb der Reaktionszone wird somit durch die Zusammensetzung der Mischung eingestellt. Beim Einsatz einer heterogenen Katalysatorschüttung ist ein Anstieg der Aktivität in Durchflußrichtung des Reaktionsgases, d. h. von der Eduktseite zur Produktseite, im Allgemeinen vorteilhaft. Die Aktivität kann längs der Durchflußrichtung des Reaktionsgases stetig ansteigen oder an einer oder mehreren Stellen treppenförmig ansteigen.

Im Allgemeinen liegt die optimierte Reaktionstemperatur für die Oxidehydrierung der n-Butene zu 1,3-Butadien unterhalb der der Oxidation von 1,3-Butadien zu Maleinsäureanhydrid. Dies gilt insbesondere dann, wenn der für die Oxidation von 1,3-Butadien zu Maleinsäureanhydrid verwendete Katalysator die Basiszusammensetzung Mo-Sb-O hat. Für den Fall eines entsprechenden Mo-V-Obasierten Katalysators kann sich bei speziellen Zusammensetzungen dieses Katalysators der Fall ergeben, daß die optimierte Reaktionstemperatur für die Oxidehydrierung der n-Butene zu 1,3-Butadien über der der Oxidation von 1,3-Butadien zu Maleinsäureanhydrid liegt.

Unter der Reaktionstemperatur wird die Temperatur der in dieser Reaktionszone befindlichen Katalysatorschüttung verstanden, welche bei Ausübung des Verfahrens in Abwesenheit einer chemischen Reaktion vorliegen würde. Ist diese Temperatur nicht an allen Stellen exakt gleich, so meint der Begriff den Zahlenmittelwert der Temperaturen längs der Reaktionszone. Sie entspricht somit in etwa der Temperatur des umgebenden Wärmeträgermediums.

Im Falle eines, in der zweiten, produktseitigen Reaktionszone befindlichen Katalysators mit der Basiszusammensetzung Mo-Sb-O, welcher geeignet ist für die Oxidation von 1,3-Butadien zu Maleinsäureanhydrid, betreibt man diesen bevorzugt bei einer um mindestens 10°C und besonders bevorzugt bei einer um mindestens 30°C höheren Reaktionstemperatur als den in der ersten, eduktseitigen Reaktionszone befindlichen Katalysator, welcher geeignet ist für die Oxidehydrierung der n-Butene zu 1,3-Butadien.

Es ist ein spezifisches Kennzeichen des erfindungsgemäßen Verfahrens, dass je nach Erfordernis, beispielsweise durch Änderung der Katalysatorbelastung oder durch Alterung der Katalysatoren die Reaktionstemperaturen der beiden Reaktionszonen unabhängig voneinander geregelt werden können.

Die Oxidehydrierung der n-Butene zu 1,3-Butadien in der ersten, eduktseitigen Reaktionszone führt man beim erfindungsgemäßen Verfahren im Allgemeinen bei einer Temperatur von 220 bis 490°C und bevorzugt von 250 bis 450°C durch.

# EP 1 417 194 B1

Die Oxidation von 1,3-Butadien zu Maleinsäureanhydrid in der zweiten, produktseitigen Reaktionszone führt man beim erfindungsgemäßen Verfahren im Allgemeinen bei einer Temperatur von 190 bis 500°C und bevorzugt von 230 bis 460°C durch.

Bezüglich des Reaktionsdruckes bestehen beim erfindungsgemäßen Verfahren im Allgemeinen keine besonderen Anforderungen. Aus praktischen Erwägungen wählt man in der Regel einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete.Gasdruck weitgehend über die gesamte Schüttung an Katalysatoren und inerten Anteilen ab.

Als n-Buten enthaltende Kohlenwasserstoffströme werden beim erfindungsgemäßen Verfahren im Allgemeinen Kohlenwasserstoffströme eingesetzt, welche einen Gesamtgehalt an n-Butenen (1-Buten, 2-trans-Buten und 2-cis-Buten) von ≥10 Gew.-% und bevorzugt von ≥30 Gew.-% enthalten. Der n-Buten enthaltende Kohlenwasserstoffstrom kann des weiteren aliphatische und aromatische, gesättigte und ungesättigte Kohlenwasserstoffe enthalten. Als Beispiele seien Methan, Ethan, Ethen, Propan, Propen, 1,3-Butadien, n-Butan, iso-Butan, iso-Buten, 1,3-Pentadien, 1,4-Pentadien, 1-Penten, 2-cis-Penten, 2-trans-Penten, n-Pentan, Cyclopentadien, Dicyclopentadien, Cyclopenten, Cyclopentan, Hexene, Hexane, Cyclohexan und Benzol genannt.

Als n-Butene enthaltende Kohlenwasserstoffströme werden beim erfindungsgemäßen Verfahren bevorzugt Ströme aus Erdgas, Steamcrackern oder FCC-Crackern eingesetzt. Besonders bevorzugt ist der sogenannte Raffinat-II-Strom, welcher aus dem Roh-$C_4$-Schnitt von Steamcrackern gewonnen wird. Unter dem Raffinat-II-Strom ist der $C_4$-Strom zu verstehen, bei dem 1,3-Butadien weitgehend abgetrennt oder durch Selektivhydrierung zu n-Butenen umgesetzt sowie iso-Buten abgetrennt wurde. Sein Gehalt an n-Butenen beträgt im Allgemeinen 50 bis 95 Gew.-%. Eine typische, nicht-limitierende Zusammensetzung eines Raffinat-II-Stroms ist in Tabelle 1 gegeben:

Tabelle 1:

| Typische Zusammensetzung eines Raffinat-II-Stroms. | |
| --- | --- |
| | Raffinat-II |
| 1-Buten | 20 bis 60 Gew.-% |
| 2-trans-Buten | 10 bis 30 Gew.-% |
| 2-cis-Buten | 5 bis 20 Gew.-% |
| n-Butan | 5 bis 35 Gew.-% |
| iso-Butan | 1 bis 10 Gew.-% |
| iso-Buten | 1 bis 2 Gew.-% |

Die Zugabe des Kohlenwasserstoffs erfolgt im allgemeinen mengengeregelt, d. h. unter stetiger Vorgabe einer definierten Menge pro Zeiteinheit. Der Kohlenwasserstoff kann in flüssiger oder gasförmiger Form dosiert werden. Bevorzugt ist die Dosierung in flüssiger Form mit anschließender Verdampfung vor Eintritt in den Rohrbündelreaktor.

Als Sauerstoff enthaltendes Gas wird beim erfindungsgemäßen Verfahren im Allgemeinen Luft, synthetische Luft, ein mit Sauerstoff angereichertes Gas oder auch sogenannter "reiner", d. h. zum Beispiel aus der Luftzerlegung stammender Sauerstoff eingesetzt. Auch das Sauerstoff-enthaltende Gas wird mengengeregelt zugegeben.

Das durch die beiden Reaktionszonen zu leitende Gas enthält im Allgemeinen Inertgas. Üblicherweise beträgt der Inertgasanteil zu Beginn 30 bis 95 Vol.-%. Inertgase sind alle Gase, welche nicht direkt zur Bildung von Maleinsäureanhydrid beitragen, wie beispielsweise Stickstoff, Wasserdampf, Edelgase, Kohlenmonoxid, Kohlendioxid, oxygenierte und nicht-oxygenierte Kohlenwasserstoffe mit weniger als vier Kohlenstoffatomen (z.B. Methan, Ethan, Propan, Methanol, Formaldehyd, Ameisensäure, Ethanol, Acetaldehyd, Essigsäure, Propanol, Propionaldehyd, Propionsäure, Acrolein, Crotonaldehyd, Acrylsäure) und deren Mischungen. Im Allgemeinen wird das Inertgas über das Sauerstoff-enthaltende Gas in das System eingebracht. Es ist aber auch möglich, weitere, zusätzliche Inertgase separat zuzuführen. So kann man das erfindungsgemäße Verfahren beispielsweise unter Zufuhr von, im Allgemeinen bis zu 50 Gew.-% Wasserdampf durchführen. Bevorzugt wird das erfindungsgemäße Verfahren ohne Zufuhr zusätzlicher Inertgase ausgeführt.

Das erfindungsgemäßen Verfahren wird im Allgemeinen im "geraden Durchgang" durchgeführt. Das eingesetzte Reaktionsgas enthält im Allgemeinen 0,1 bis 30 Vol.-% und bevorzugt 0,5 bis 7 Vol.-% n-Butene und 5 bis 50 Vol.-%, bevorzugt 10 bis 40 Vol.-% Sauerstoff. Der Umsatz an n-Butenen beträgt in der Regel 50 bis 100 %, bevorzugt 75 bis 100 % und besonders bevorzugt 85 bis 100 %. Die nicht umgesetzten n-Butene können nach Abtrennung des gebildeten Maleinsäureanhydrids und weiterer Produkte wieder in den Eingangs-Feed zurückgeführt werden. Bevorzugt wird bei einem sehr hohen n-Buten-Umsatz im geraden Durchgang ohne Rückführung von nicht umgesetzten n-Butenen gearbeitet.

Die Abtrennung von Maleinsäureanhydrid kann beispielsweise durch Absorption in einem geeigneten Absorptionsmittel erfolgen. Geeignete Absorptionsmittel sind beispielsweise Wasser oder organische Lösungsmittel. Bei Absorption in Wasser wird Maleinsäureanhydrid zu Maleinsäure hydratisiert. Bevorzugt ist die Absorption in einem organischen Lösungsmittel. Geeignete organische Lösungsmittel sind beispielsweise die in WO 97/43242 genannten, hochsiedenden Lösungsmittel, wie Trikresylphosphat, Dibutylmaleat, hochmolekulares Wachs, aromatische Kohlenwasserstoffe mit einem Siedepunkt über 140°C oder Di-$C_4$-$C_8$-alkylphthalate, wie etwa Dibutylphthalat. In den genannten Lösungsmitteln werden im allgemeinen auch oxygenierte Kohlenwasserstoff-Nebenprodukte absorbiert. Die Absorption kann beispielsweise bei einer Temperatur von 60 bis 160°C und einem Druck von 0,1 bis 0,5 MPa abs oder darüber durchgeführt werden. Geeignete Verfahrensweisen sind etwa die Durchleitung des gasförmigen, gegebenenfalls abgekühlten Reaktoraustrags durch einen mit Absorptionsflüssigkeit gefüllten Behälter oder das Versprühen der Absorptionsflüssigkeit im Gasstrom. Entsprechende Methoden zum.Auswaschen von Gasströmen sind dem Fachmann bekannt.

Des weiteren wurde die Verwendung eines Rohrbündelreaktors mit zwei aufeinanderfolgenden Reaktionszonen mit mindestens einem Wärmeträgerkreislauf im Bereich der ersten, eduktseitigen Reaktionszone und mindestens einem weiteren Wärmeträgerkreislauf im Bereich der zweiten, produktseitigen Reaktionszone in der heterogenkatalytischen Gasphasenoxidation eines n-Buten enthaltenden Kohlenwasserstoffstroms mit Sauerstoff zu Maleinsäureanhydrid, gefunden.

Bevorzugt ist die Verwendung eines Rohrbündelreaktors mit zwei aufeinanderfolgenden Reaktionszonen mit einem Wärmeträgerkreislauf im Bereich der ersten, eduktseitigen Reaktionszone und einem weiteren Wärmeträgerkreislauf im Bereich der zweiten, produktseitigen Reaktionszone in der heterogenkatalytischen Gasphasenoxidation eines n-Buten enthaltenden Kohlenwasserstoffstroms mit Sauerstoff zu Maleinsäureanhydrid.

Im Folgenden ist eine bevorzugte Ausführungsform zur Herstellung von Maleinsäureanhydrid durch heterogenkatalytische Gasphasenoxidation eines n-Butene enthaltenden Kohlenwasserstoffstroms mit einem Sauerstoff enthaltenden Gas in einem Rohrbündelreaktor mit zwei aufeinanderfolgenden Reaktionszonen mit einem Wärmeträgerkreislauf im Bereich der ersten, eduktseitigen Reaktionszone und einem weiteren Wärmeträgerkreislauf im Bereich der zweiten, produktseitigen Reaktionszone, beschrieben.

Beide Wärmeträgerkreisläufe des Rohrbündelreaktors werden mit dem Wäremträgermedium von unten nach oben durchströmt. Der Rohrbündelreaktor wird in Gleichstromfahrweise betrieben, d. h. das Reaktionsgas (Eduktgas) wird von unten zugeführt. Als Reaktionsgas wird ein Gasgemisch aus Raffinat-11 in Luft mit einem n-Butene-Gehalt von 0,5 bis 7 Vol.-%. Von unten nach oben gesehen enthält der Rohrbündelreaktor in der untersten Schüttungszone, welche als Vorheizzone fungiert, Inertmaterial. In der folgenden, ersten Reaktionszone befindet sich der Katalysator zur Oxidehydrierung der n-Butene zu 1,3-Butadien, welcher als Aktivmasse ein Mo-Bi-Fe-0-haltiges Multimetalloxidsystem enthält. Die erste Reaktionszone wird in einem Temperaturbereich von 250 bis 450°C betrieben. Im Bereich oder in der Nähe der im Rohrbündelreaktor befindlichen Trennplatte zur Abtrennung beider Wärmeträgerkreisläufe befindet sich eine inerte Zwischenschicht mit einem Leerraumanteil im Bereich 40 bis 99,5%. In der folgenden, zweiten Reaktionszone befindet sich der Katalysator zur Oxidation von 1,3-Butadien zu Maleinsäureanhydrid, welcher als Aktivmasse ein Mo-Sb-O-haltiges oder ein Mo-V-O-haltiges Multimetalloxidsystem enthält. Die zweite Reaktionszone wird in einem Temperaturbereich von 230 bis 460°C betrieben. Der Druck des Reaktionsgases am Reaktoreingang liegt im Bereich von 0,01 bis 0,5 MPa Überdruck. Temperatur und Katalysatorbelastung werden im Allgemeinen derart gewählt so dass ein Gesamtumsatz an n-Butenen von ≥ 50 %, bevorzugt ≥ 75% und besonders bevorzugt ≥ 85% resultiert. Das am Reaktorkopf entnommene Reaktionsgas wird zur Abtrennung des gebildeten Maleinsäureanhydrids einer Absorptionsstufe zugeführt.

Das erfindungsgemäße Verfahren ermöglicht die Herstellung von Maleinsäureanhydrid durch heterogenkatalytische Gasphasenoxidation unter Einsatz eines preiswerten und gut verfügbaren Kohlenwasserstoffstroms bei einer hohen Kohlenwasserstoff-Belastung des Katalysators, einem hohen Umsatz, einer hohen Selektivität sowie einer hohen Ausbeute an Wertprodukt und somit einer hohe Raum-Zeit-Ausbeute. Aufgrund der beiden Wärmeträgerkreisläufe, die eine getrennte Temperaturführung der beiden Reaktionszonen erlauben, gestattet das erfindungsgemäße Verfahren eine flexible Reaktionsführung, welche auch bei Schwankungen in der Menge, Qualität oder Reinheit der Ausgangsstoffe oder bei einer fortschreitenden Katalysatordesaktivierung eine hohe Raum-Zeit-Ausbeute über einen langen Zeitraum hinweg ermöglicht. Die Realisierung des Verfahrens in einem einzigen Rohrbündelreaktor mit zwei aufeinanderfolgenden Reaktionszonen führt gegenüber einer Realisierung in zwei hintereinandergeschalteten Rohrbündelreaktoren zu einer deutlichen Einsparung an Investitionskosten und zu einer signifikanten Vereinfachung der Gesamtanlage.

Die bei einer bevorzugten Variante des erfindungsgemäßen Verfahrens vorhandene inerte Zwischenschicht zwischen der ersten, eduktseitigen Reaktionszone und der zweiten, produktseitige Reaktionszone ermöglicht zudem die Abscheidung höhermolekularer Nebenprodukte aus der ersten Reaktionszone, wie beispielsweise Oligomere, Polymere oder Koks und wirkt einer Ablagerung am Katalysator der zweiten Reaktionszone und somit einem allmählichen Anstieg des Druckverlustes durch derartige Ablagerungen am Katalysator entgegen.

Das gewonnene Maleinsäureanhydrid kann beispielsweise weiterverarbeitet werden zu γ-Butyrolacton, Tetrahydrofuran, 1,4-Butandiol oder Gemischen davon. Geeignete Verfahren sind dem Fachmann bekannt. Der Vollständigkeit halber sei auf die beiden Schriften WO 97/43234 (direkte Hydrierung von Maleinsäureanhydrid in der Gasphase) und WO 97/43242 (Hydrierung eines Maleinsäurediesters in der Gasphase) verwiesen.

Beispiele

Definitionen

Die in dieser Schrift verwendeten Größen sind, falls nicht anders erwähnt, wie folgt definiert:

$$\text{Umsatz } U = \frac{n_{KW,Reaktor,ein} - n_{KW,Reaktor,aus}}{n_{KW,Reaktor,ein}}$$

$$\text{Selektivität } S_{MSA} = \frac{n_{MSA,Reaktor,aus}}{n_{KW,Reaktor,ein} - n_{KW,Reaktor,aus}}$$

$$\text{Ausbeute } A_{MSA} = U \cdot S_{MSA}$$

| | |
|---|---|
| $U$ | Umsatz an Kohlenwasserstoffen pro Reaktordurchgang |
| $S_{MSA}$ | Selektivität bzgl. Maleinsäureanhydrid pro Reaktordurchgang |
| $A_{MSA}$ | Ausbeute an Maleinsäureanhydrid pro Reaktordurchgang |
| $n_{KW}$, Reaktor, ein | Stoffmengenstrom an Kohlenwasserstoffen am Reaktoreingang [Mol/h] |
| $n_{KW}$, Reaktor, aus | Stoffmengenstrom an Kohlenwasserstoffen am Reaktorausgang [Mol/h] |
| $n_{KW}$, Anlage, ein | Stoffmengenstrom an Kohlenwasserstoffen am Eingang der Anlage [Mol/h] |
| $n_{KW}$, Anlage, aus | Stoffmengenstrom an Kohlenwasserstoffen am Ausgang der Anlage [Mol/h] |
| $n_{MSA}$, Reaktor, aus | Stoffmengenstrom an Maleinsäureanhydrid am Reaktorausgang [mol/h] |
| $n_{MSA}$, Anlage, aus | Stoffmengenstrom an Maleinsäureanhydrid am Ausgang der Anlage [Mol/h] |

Herstellung des Oxidehydrierkatalysators K1

In einem beheizbaren 10 L-Rührbehälter aus Glas wurden 1750,9 g wässrige Kobaltnitrat-Lösung mit einem freien $HNO_3$-Gehalt von 0,2 Gew.-% und einem Co-Gehalt von 12,5 Gew.-% (= 3,71 Mol Co) vorgelegt. In der vorgelegten Kobaltnitrat-Lösung wurden unter Rühren 626,25 g festes $Fe(NO_3)_3 \cdot 9H_2O$ mit einem Fe-Gehalt von 14,2 Gew.-% (= 1,59 Mol Fe) bei Raumtemperatur gelöst. In die erhaltene Lösung wurden bei Raumtemperatur 599,5 g Bismutnitrat-Lösung mit einem freien $HNO_3$-Gehalt von 3 Gew.-% und einem Bi-Gehalt von 11,1 Gew.-% (= 0,32 Mol Bi) zugegeben. Anschließend wurden 106,23 g festes $Cr(NO_3)_3 \cdot 9 H_2O$ (= 0,27 Mol Cr) zugegeben. Nach dem Aufheizen auf 60°C und weiterem Rühren ergab sich eine rote Lösung (Lösung A).

In einem weiteren beheizbaren 3 L-Rührbehälter aus Glas wurden 2000 mL Wasser vorgelegt. Anschließend wurden 2,38 g KOH (= 0,042 Mol K) und 1124,86 g $(NH_4)_6Mo_7O_{24} \cdot 4 H_2O$ (= 6,37 Mol Mo) zugegeben und bei 60°C gelöst. Die erhaltene Lösung zeigte eine leichte Trübung (Lösung B).

Anschließend wurde Lösung B in Lösung A gepumpt, wobei letztere gerührt wurde. In die erhaltene, 60°C heiße, dunkelgelbe Suspension wurden 102,05 g $SiO_2$-Sol mit einem $SiO_2$-Gehalt von 50 Gew.-% ("Ludox TM", Fa. DuPont) (= 0,85 Mol Si ) zugegeben.

Die erhaltene Suspension wurde bei 60°C 30 Minuten lang nachgerührt und anschließend sprühgetrocknet (Eingangstemperatur 370°C, Ausgangstemperatur 110 bis 112°C). Das erhaltene Sprühpulver wurde mit 4 Gew.-% Graphit versetzt und anschließend zu Volltabletten mit einem Durchmesser von 5 mm und einer Höhe von 3 mm tablettiert. Die Volltabletten wurden 6 Stunden lang bei 480°C in einem Muffelofen auf einem mit Luft durchströmten Drahtsieb (Maschenweite 3,5 mm) und einer durchströmenden Luftmenge von 100 L/h getempert. Die kalzinierten Tabletten wurden über ein Drahtsieb zu einem Katalysatorsplitt K1 mit einem durchschnittlichen Granulatdurchmesser von 2 bis 3 mm zerkleinert.

Der Oxidehydrierkatalysators K1 hatte die nominale Zusammensetzung $Mo_{12}Bi_{0,6}Fe_3Co_7Cr_{0,5}Si_{1,6}K_{0,08}O_x$.

Herstellung des Oxidationskatalysators K2

In einem beheizbaren 10 L-Rührbehälter aus Glas wurden 2500 mL Wasser vorgelegt und 226,72 g $MoO_3$ (Fa. Fluka) (= 1,575 Mol Mo), 25,17 g $TiO_2$ ("Finn Ti S 150", Fa. Kemira) (= 0,315 Mol Ti), 6,99 g $Nb_2O_5$ (Fa. H.C. Starck) (= 0,052 Mol Nb) sowie 21,7 g $SnC_2O_4$ (Fa. Merck) (= 0,105 Mol Sn) gegeben. Die erhaltene Supension wurde 2 Stunden unter Rückfluß gekocht. Nach der Zugabe von 76,52 g $Sb_2O_3$ (Fa. Merck) (= 0,52 Mol Sb) wurde die Suspension weitere 16 Stunden lang unter Rückfluß gekocht und anschließend auf 50°C abgekühlt. Danach wurden 35 g einer wässrigen Acronal-Lösung mit einem Acronal-Gehalt von 50 Gew.-% (wasserlösliches Polymer auf Acrylsäure-Basis, Fa. BASF) und danach 140 g Formamid (Fa. BASF) zugegeben.

Zur Herstellung eines Schalenkatalysators wurde die resultierende Suspension über eine Zweistoffdüse auf 750 g Steatit-Kugeln mit einem mittleren Durchmesser von 2,5 bis 3,2 mm (Fa. Ceramtec) gesprüht. Während der Beschichtung wurden die Steatit-Kugeln in einer auf 150°C temperierten Beschichtungstrommel mit einem inneren Durchmesser von 300 mm bewegt, wobei sich die Beschichtungstrommel mit 60 Umdrehungen pro Minute drehte. Es resultierte ein Schalenkatalysator K2 mit einem Aktivmassen-Anteil von 31 Gew.-%.

Der Oxidationskatalysator hatte die nominale Zusammensetzung $SbMo_{3,06}Ti_{0,6}Nb_{0,1}Sn_{0,2}O_x$.

Versuchsanlage V1

Die Versuchsanlage war ausgestattet mit einer Zufuhr-Einheit und einem Reaktorrohr mit zwei getrennt regelbaren, elektrisch betriebenen Heizzonen. Der Ersatz eines, über Wärmeträgerkreisläufe temperierten Rohrbündelreaktors durch ein Reaktorrohr mit elektrisch betriebenen Heizzonen ist im Labor- und Technikumsmaßstab sehr gut möglich.

Das eingesetzte Reaktorrohr hatte eine Länge von 2 m und einen inneren Durchmesser von 12 mm. Das stehend betriebene Reaktorrohr wurde von unten nach oben durchströmt. Das untere Ende des Reaktorrohres war zur Auflage der Schüttungen mit einem Netz versehen. Das Reaktorrohr enthielt von unten nach oben gesehen folgende fünf Schüttungszonen:

| | |
|---|---|
| Schüttungszone A (unten) | 15 cm Steatit-Kugeln mit einem mittleren Durchmesser von 2 bis 3 mm. |
| Schüttungszone B | 80 cm Oxidehydrierkatalysators K1. |
| Schüttungszone C | 30 cm Steatit-Kugeln mit einem mittleren Durchmesser von 2 bis 3 mm. |
| Schüttungszone D | 65 cm Oxidationskatalysators K2. |
| Schüttungszone E (oben) | 10 cm Steatit-Kugeln mit einem mittleren Durchmesser von 2 bis 3 mm. |

Die Schüttungszonen A und B befanden sich in der ersten Heizzone und wurden auf die Temperatur $T_1$ temperiert, wobei die Schüttungszone A als Vorheizzone für die Oxidehydrierungszone fungierte. Die Schüttungszonen C, D und E befanden sich im Wesentlichen in der zweiten Heizzone und wurden auf die Temperatur $T_2$ temperiert, wobei die Schüttungszone C ("inerte Zwischenschicht") als Vorheizzone für die Oxidationszone fungierte und die Abscheidung höhermolekularer Nebenprodukte aus der ersten Reaktionszone ermöglichte.

Die Versuchsanlage wurde im geraden Durchgang betrieben. Aus dem am oberen Ende des Reaktors abströmende Reaktionsgas wurde gaschromatographisch analysiert.

Als Eduktgasstrom wurde ein Gemisch aus 2 Vol.-% n-Butene (Gemisch aus 60 % 1-Buten und 40 % 2-Butene) in Luft zugeführt. Die Menge an zugeführtem Eduktgas betrug 145 NL/h, was einer GHSV von 1600 NL/[(L Katalysator K1) · h] entspricht. Der Druck am oberen Reaktorausgang betrug 0,01 MPa Überdruck.

Beispiel 1 (erfindungsgemäß)

In Beispiel 1 wurden die für beide Reaktionen optimalen Reaktionstemperaturen eingestellt. Die Oxidehydrierung an K1 wurde bei $T_1$ = 330°C und die Oxidation an K2 bei $T_2$ = 400°C durchgeführt. Die in Tabelle 2 dargestellten Werte wurden nach einer Einfahrzeit von 3 Tagen bestimmt.

Beispiel 2 (Vergleichsbeispiel)

In Beispiel 2 wurde für beide Reaktionszonen die für die Oxidehydrierung an K1 optimale Temperatur eingestellt. $T_1$ und $T_2$ betrugen somit 330°C. Die in Tabelle 2 dargestellten Werte wurden nach einer Einfahrzeit von 3 Tagen bestimmt.

Beispiel 3 (Vergleichsbeispiel)

In Beispiel 3 wurde für beide Reaktionszonen die für die Oxidation an K2 optimale Temperatur eingestellt. $T_1$ und $T_2$ betrugen somit 400°C. Die in Tabelle 2 dargestellten Werte wurden nach einer Einfahrzeit von 3 Tagen bestimmt.

Tabelle 2:

| Ergebnisse der Beispiele 1 bis 3 | | | | |
|---|---|---|---|---|
| Beispiel | $T_1$ [°C] | $T_2$ [°C] | U [%] | $A_{MSA}$ [%] |
| 1 | 330 | 400 | 99 | 62 |
| 2* | 330 | 330 | 99 | 32 |
| 3* | 400 | 400 | 99 | 54 |

\* Vergleichsbeispiel

Das erfindungsgemäße Beispiel 1 zeigt, dass durch eine optimale Temperierung der zwei aufeinanderfolgenden Reaktionszonen, wie sie in einem Rohrbündelreaktor mit einem Wärmeträgerkreislauf im Bereich der ersten, eduktseitigen Reaktionszone und einem weiteren Wärmeträgerkreislauf im Bereich der zweiten, produktseitigen Reaktionszone ermöglicht werden kann, eine deutliche höhere Ausbeute an Maleinsäureanhydrid erzielt wird als in den beiden Vergleichsbeispielen mit ein und derselben Temperatur für beide Reaktionszonen.

**Patentansprüche**

1. Verfahren zur Herstellung von Maleinsäureanhydrid durch heterogenkatalytische Gasphasenoxidation eines n-Buten enthaltenden Kohlenwasserstoffstroms mit Sauerstoff enthaltenden Gasen in einem Rohrbündelreaktor mit zwei aufeinanderfolgenden Reaktionszonen, wobei die erste, eduktseitige Reaktionszone mindestens einen Katalysator enthält, welcher geeignet ist für die Oxidehydrierung der n-Butene zu 1,3-Butadien und die zweite, produktseitige Reaktionszone mindestens einen Katalysator enthält, welcher geeignet ist für die Oxidation von 1,3-Butadien zu Maleinsäureanhydrid, **dadurch gekennzeichnet, dass** man einen Rohrbündelreaktor einsetzt, welcher mindestens einen Wärmeträgerkreislauf im Bereich der ersten, eduktseitigen Reaktionszone und mindestens einen weiteren Wärmeträgerkreislauf im Bereich der zweiten, produktseitigen Reaktionszone aufweist.

2. Verfahren nach Anspruch 1, **dadurch gekennzeichnet, dass** man das Reaktionsgas aus der ersten, eduktseitigen Reaktionszone vor Eintritt in die zweite, produktseitige Reaktionszone durch eine inerte Zwischenschicht leitet.

3. Verfahren nach Anspruch 2, **dadurch gekennzeichnet, dass** man als inerte Zwischenschicht ein inertes Material mit einem Leerraumvolumen von 40 bis 99,5% einsetzt.

4. Verfahren nach den Ansprüchen 1 bis 3, **dadurch gekennzeichnet, dass** man als Aktivmasse des für die Oxidehydrierung der n-Butene zu 1,3-Butadien geeigneten Katalysators ein Multimetalloxid der Stöchiometrie der allgemeinen Formel (I)

$$Mo_{12}Bi_aFe_bCo_cNi_dCr_eX^1_fKgO, \qquad (I),$$

in der die Variablen nachfolgende Bedeutung aufweisen:

$X^1$ = W, Sn, Mn, La, Ce, Ge, Ti, Zr, Hf, Nb, P, Si, Sb, Al, Cd und/oder Mg;
a = 0,5 bis 5;
b = 0 bis 5;
c = 0 bis 10;
d = 0 bis 10;
e = 0 bis 10;
f = 0 bis 5;
g = 0 bis 2; und

x = eine Zahl, die durch die Wertigkeit und Häufigkeit der von Sauerstoff verschiedenen Elemente in (I) bestimmt wird;

einsetzt.

5. Verfahren nach den Ansprüchen 1 bis 4, **dadurch gekennzeichnet, dass** man als Aktivmasse des für die Oxidation von 1,3-Butadien zu Maleinsäureanhydrid geeigneten Katalysators ein. Multimetalloxid der Stöchiometrie der allgemeinen Formel (II)

$$SbMo_aSn_bX^2{}_cO_x \qquad (II),$$

in der die Variablen nachfolgende Bedeutung aufweisen:

$X^2$ = V, Fe, Ni, Li, Ce, Nb, Ta, W, Ti, Zr, B, P, Al und/oder Mg;
a = 1 bis 10;
b = 0 bis 5;
c = 0 bis 3; und
x = eine Zahl, die durch die Wertigkeit und Häufigkeit der von Sauerstoff verschiedenen Elemente in (II) bestimmt wird;

einsetzt.

6. Verfahren nach den Ansprüchen 1 bis 4, **dadurch gekennzeichnet, dass** man als Aktivmasse des für die Oxidation von 1,3-Butadien zu Maleinsäureanhydrid geeigneten Katalysators ein Multimetalloxid der Stöchiometrie der allgemeinen Formel (III)

$$Mo_{12}V_aW_bX^3{}_cO_x \qquad (III),$$

in der die Variablen nachfolgende Bedeutung aufweisen:

$X^3$ = La, Mn, Fe, Cu, Al, Co, Ni, Bi, Ag, P, Zn, Cd, As, Cr, Sn, U, Ti, Nb, Ge, Alkali und/oder Erdalkali;
a = 0,1 bis 12;
b = 0 bis 5;
c = 0 bis 12; und
x = eine Zahl, die durch die Wertigkeit und Häufigkeit der von Sauerstoff verschiedenen Elemente in (III) bestimmt wird;

einsetzt.

7. Verfahren nach den Ansprüchen 1 bis 6, **dadurch gekennzeichnet, dass** man die Oxidehydrierung der n-Butene zu 1,3-Butadien in der ersten, eduktseitigen Reaktionszone bei einer Temperatur von 220 bis 490°C und die Oxidation von 1,3-Butadien zu Maleinsäureanhydrid in der zweiten, produktseitigen Reaktionszone bei einer Temperatur von 190 bis 500°C durchführt.

8. Verfahren nach den Ansprüchen 1 bis 7, **dadurch gekennzeichnet, dass** man einen Reaktoreingangsdruck von 0,005 bis 1 MPa Überdruck wählt.

9. Verfahren nach den Ansprüchen 1 bis 8, **dadurch gekennzeichnet, dass** man als n-Buten enthaltenden Kohlenwasserstoffstrom einen Raffinat-II-Strom einsetzt.

10. Verwendung eines Rohrbündelreaktors gemäss eines Verfahrens nach Anspruch 1 mit zwei aufeinanderfolgenden Reaktionszonen mit mindestens einem Wärmeträgerkreislauf im Bereich der ersten, eduktseitigen Reaktionszone und mindestens einem weiteren Wärmeträgerkreislauf im Bereich der zweiten, produktseitigen Reaktionszone in der heterogenkatalytischen Gasphasenoxidation eines n-Buten enthaltenden Kohlenwasserstoffstroms mit Sauerstoff zu Maleinsäureanhydrid.

**Claims**

1. A process for preparing maleic anhydride by heterogeneously catalyzed gas-phase oxidation of an n-butene-containing hydrocarbon stream by means of oxygen-containing gases in a shell-and-tube reactor having two successive reaction zones, where the first, feed-side reaction zone contains at least one catalyst which is suitable for the oxydehydrogenation of n-butenes to 1,3-butadiene and the second, product-side reaction zone contains at least one catalyst which is suitable for the oxidation of 1,3-butadiene to maleic anhydride, wherein the shell-and-tube reactor has at least one heat transfer medium circuit in the region of the first, feed-side reaction zone and at least one further heat transfer medium circuit in the region of the second, product-side reaction zone.

2. A process as claimed in claim 1, wherein the reaction gas from the first, feed-side reaction zone is passed through an inert intermediate bed before it enters the second, product-side reaction zone.

3. A process as claimed in claim 2, wherein the inert intermediate bed used is a bed of inert material having an empty volume fraction of from 40 to 99.5%.

4. A process as claimed in any of claims 1 to 3, wherein the active composition of the catalyst suitable for the oxydehydrogenation of n-butenes to 1,3-butadiene is a multimetal oxide of the formula (I)

$$Mo_{12}Bi_aFe_bCo_cNi_dCr_eX^1_fK_gO_x \qquad \text{(I)},$$

where the variables have the following meanings:

$X^1 =$  W, Sn, Mn, La, Ce, Ge, Ti, Zr, Hf, Nb, P, Si, Sb, Al, Cd and/or Mg;
$a =$  0.5 to 5;
$b =$  0 to 5;
$c =$  0 to 10;
$d =$  0 to 10;
$e =$  0 to 10;
$f =$  0 to 5;
$g =$  0 to 2; and
$x =$  a number determined by the valence and abundance of the elements other than oxygen in (I).

5. A process as claimed in any of claims 1 to 4, wherein the active composition of the catalyst suitable for the oxidation of 1,3-butadiene to maleic anhydride is a multimetal oxide of the formula (II)

$$SbMo_aSn_bX^2_cO_x \qquad \text{(II)},$$

where the variables have the following meanings:

$X^2 =$  V, Fe, Ni, Li, Ce, Nb, Ta, W, Ti, Zr, B, P, Al and/or Mg;
$a =$  1 to 10;
$b =$  0 to 5;
$c =$  0 to 3; and
$x =$  a number determined by the valence and abundance of the elements other than oxygen in (II).

6. A process as claimed in any of claims 1 to 4, wherein the active composition of the catalyst suitable for the oxidation of 1,3-butadiene to maleic anhydride is a multimetal oxide of the formula (III)

$$MO_{12}V_aW_bX^3_cO_x \qquad \text{(III)},$$

where the variables have the following meanings:

$X^3 =$  La, Mn, Fe, Cu, Al, Co, Ni, Bi, Ag, P, Zn, Cd, As, Cr, Sn, U, Ti, Nb, Ge, an alkali metal and/or an alkaline earth metal;

a = 0.1 to 12;

b = 0 to 5;

c = 0 to 12; and

x = a number determined by the valence and abundance of the elements other than oxygen in (III).

**7.** A process as claimed in any of claims 1 to 6, wherein the oxydehydrogenation of n-butenes to 1,3-butadiene in the first, feed-side reaction zone is carried out at from 220 to 490°C and the oxidation of 1,3-butadiene to maleic anhydride in the second, product-side reaction zone is carried out at from 190 to 500°C.

**8.** A process as claimed in any of claims 1 to 7, wherein the pressure at the reactor inlet is from 0.005 to 1 MPa gauge pressure.

**9.** A process as claimed in any of claims 1 to 8, wherein the n-butene-containing hydrocarbon stream used is a raffinate II stream.

**10.** The use of a shell-and-tube reactor as per a process as claimed in claim 1 having two successive reaction zones and at least one heat transfer medium circuit in the region of the first, feed-side reaction zone and at least one further heat transfer medium circuit in the region of the second, product-side reaction zone in the heterogeneously catalyzed gas-phase oxidation of an n-butene-containing hydrocarbon stream by means of oxygen to form maleic anhydride.

**Revendications**

**1.** Procédé de préparation d'anhydride maléique par oxydation en phase gazeuse à catalyse hétérogène d'un courant d'hydrocarbure contenant du n-butène avec des gaz contenant de l'oxygène, dans un réacteur à faisceau tubulaire présentant deux zones réactionnelles successives, dans lequel la première zone réactionnelle du côté matière de départ contient au moins un catalyseur qui est approprié pour l'oxydéshydrogénation des n-butènes en 1,3-butadiène et la deuxième zone réactionnelle du côté produit contient au moins un catalyseur qui est approprié pour l'oxydation de 1,3-butadiène en anhydride maléique, **caractérisé en ce qu'**on met en oeuvre un réacteur à faisceau tubulaire qui présente au moins un circuit caloporteur dans la région de la première zone réactionnelle du côté matière de départ et au moins un autre circuit caloporteur dans la région de la deuxième zone réactionnelle du côté produit.

**2.** Procédé suivant la revendication 1, **caractérisé en ce qu'**on conduit le gaz réactionnel issu de la première zone réactionnelle du côté matière de départ au travers d'une couche intermédiaire inerte avant l'entrée dans la deuxième zone réactionnelle du côté produit.

**3.** Procédé suivant la revendication 2, **caractérisé en ce que**, comme couche intermédiaire inerte, on met en oeuvre une matière inerte présentant un volume d'espace libre de 40 à 99,5%.

**4.** Procédé suivant l'une des revendications 1 à 3, **caractérisé en ce que**, comme masse active du catalyseur approprié pour l'oxydéshydrogénation des n-butènes en 1,3-butadiène, on met en oeuvre un oxyde multimétallique présentant la stoechiométrie de la formule générale (I) :

$$Mo_{12}Bi_aFe_bCo_cNi_dCr_eX^1{}_fK_gO_x \qquad (I)$$

dans laquelle les variables ont la signification suivante :

$X^1$ = W, Sn, Mn, La, Ce, Ge, Ti, Zr, Hf, Nb, P, Si, Sb, Al, Cd et/ou Mg,

a = 0,5 à 5,

b = 0 à 5,

c = 0 à 10,

d = 0 à 10,

e = 0 à 10,

f = 0 à 5,

g = 0 à 2, et

x = un nombre qui est déterminé par la valence et la fréquence des éléments dans (I) qui sont différents de l'oxygène.

5. Procédé suivant l'une des revendications 1 à 4, **caractérisé en ce que**, comme masse active du catalyseur approprié pour l'oxydation de 1,3-butadiène en anhydride maléique, on met en oeuvre un oxyde multimétallique présentant la stoechiométrie de la formule générale (II) :

$$SbMO_aSn_bX^2{}_cO_x \qquad (II)$$

dans laquelle les variables ont la signification suivante :

$X^2$ = V, Fe, Ni, Li, Ce, Nb, Ta, W, Ti, Zr, B, P, Al et/ou Mg,

a = 1 à 10,

b = 0 à 5,

c = 0 à 3, et

x = un nombre qui est déterminé par la valence et la fréquence des éléments dans (II) qui sont différents de l'oxygène.

6. Procédé suivant l'une des revendications 1 à 4, **caractérisé en ce que**, comme masse active du catalyseur approprié pour l'oxydation de 1,3-butadiène en anhydride maléique, on met en oeuvre un oxyde multimétallique présentant la stoechiométrie de la formule générale (III) :

$$MO_{12}V_aW_bX^3{}_cO_x \qquad (III)$$

dans laquelle les variables ont la signification suivante :

$X^3$ = La, Mn, Fe, Cu, Al, Co, Ni, Bi, Ag, P, Zn, Cd, As, Cr, Sn, U, Ti, Nb, Ge, métal alcalin et/ou alcalino-terreux,

a = 0,1 à 12,

b = 0 à 5,

c = 0 à 12, et

x = un nombre qui est déterminé par la valence et la fréquence des éléments dans (III) qui sont différents de l'oxygène.

7. Procédé suivant l'une des revendications 1 à 6, **caractérisé en ce qu'**on effectue l'oxydéshydrogénation des n-butènes en 1,3-butadiène dans la première zone réactionnelle du côté matière de départ à une température de 220 à 490°C et l'oxydation de 1,3-butadiène en anhydride maléique dans la deuxième zone réactionnelle du côté produit à une température de 190 à 500°C.

8. Procédé suivant l'une des revendications 1 à 7, **caractérisé en ce qu'**on sélectionne une pression d'entrée du réacteur de 0,005 à 1 MPa de surpression.

9. Procédé suivant l'une des revendications 1 à 8, **caractérisé en ce que**, comme courant d'hydrocarbure contenant du n-butène, on met en oeuvre un courant de raffinat-II.

10. Utilisation d'un réacteur à faisceau tubulaire conformément à un procédé suivant la revendication 1, qui présente deux zones réactionnelles. successives avec au moins un circuit caloporteur dans la région de la première zone réactionnelle du côté matière de départ et au moins un autre circuit caloporteur dans la région de la deuxième zone réactionnelle du côté produit, dans l'oxydation en phase gazeuse à catalyse hétérogène d'un courant d'hydrocarbure contenant du n-butène par de l'oxygène pour former de l'anhydride maléique.